# EUROPÄISCHE PATENTSCHRIFT

(11) **EP 0 536 369 B1**
(45) Veröffentlichungstag und Bekanntmachung des Hinweises auf die Patenterteilung: **15.11.1995**
(21) Anmeldenummer: 92909116.3
(22) Anmeldetag: 27.04.1992
(51) Int. Cl.: C07C 59/01, C07C 235/08, C07C 215/10, A61K 31/205, A61K 31/16

(54) **DERIVATE DER 4-HYDROXY-BUTTERSÄURE**
4-HYDROXY BUTYRIC ACID DERIVATIVES
DERIVES DE L'ACIDE 4-HYDROXYBUTYRIQUE

(30) Priorität: 29.04.1991 DE 4113984
(43) Veröffentlichungstag der Anmeldung: 14.04.1993
(73) Patentinhaber: Dr. Franz Köhler Chemie GmbH, D-64665 Alsbach-Hähnlein (DE)
(72) Erfinder: KÖHLER, Gernot, D-6146 Alsbach-Hähnlein 1 (DE); KÖHLER, Anita, D-6146 Alsbach-Hähnlein 1 (DE)
(74) Vertreter: Zinngrebe, Horst, Dr.rer.nat.
(86) Internationale Anmeldenummer: DE9200336
(87) Internationale Veröffentlichungsnummer: WO9219581

(56) Entgegenhaltungen:
- US-A- 4 143 159
- US-A- 4 549 010
- S. BUDAVARI et al: "The Merck Index, an Encyclopedia of Chemicals, Drugs, and Biologicals", 11. Auflage, 1989, Merck & Co., Inc., Rahway, NJ, US, Seite 1365, Absatz 8603

## Beschreibung

Die Erfindung betrifft wasserlösliche Derivate der 4-Hydroxy-Buttersäure, Verfahren zu ihrer Herstellung und ihre pharmakologische Verwendung.

Die 4-Hydroxy-Buttersäure oder auch gamma-Hydroxybuttersäure (GHB) ist seit vielen Jahren als Narkotikum allgemein in klinischer Anwendung. Als Pharmazeutikum ist diese Substanz ausschließlich in Form des Natrium-Salzes erhältlich und z.B. in Deutschland unter dem Warenzeichen SOMSANIT zugelassen. Alle experimentellen und klinischen Untersuchungen wurden ohne Ausnahme mit dem Natrium-Salz der GHB, der Säure selbst bzw. dem entsprechenden Lakton, durchgeführt.

ROBERTS und FRANKEL haben 1950 im Säugetierhirn gamma-Aminobuttersäure (GABA) nachgewiesen. Zwei Jahre später hat FLOREY die inhibierende Wirkung der GABA auf das Zentralnervensystem entdeckt, die zu den Symptomen des physiologischen Schlafes führt. ROBERTS und seine Mitarbeiter sowie ALBERS und SALVADOR haben 1958 mitgeteilt, daß gamma-Aminobuttersäure im Gehirn durch eine spezifische Transaminase in den Bernsteinsäurehalbaldehyd und dieser durch eine Dehydrogenase zu GHB reduziert wird.

Parenteral applizierte gamma-Aminobuttersäure vermag die Blut-Hirn-Schranke nicht zu passieren. LABORIT und Mitarbeiter, BESSMAN und FISHBEIN haben nach Derivaten gesucht, die auf hämatogenem Wege das Zentralnervensystem erreichen. Im Rahmen dieser Untersuchungen haben LABORIT, JOUANY, GERARD und FABIAN 1960 erstmals über die narkotische Wirkung der 4-Hydroxybuttersäure berichtet.

Quantitative Studien von BESSMAN und FISHBEIN über die Verteilung der GHB und des korrespondierenden gamma-Butyrolactons im Organismus bestätigen, daß GHB ein physiologisches Stoffwechselprodukt des menschlichen Gehirns ist, wo dieser wahrscheinlich einzige Metabolit mit anaesthetischen Wirkungen eine Konzentration bis zu 0,3 mmol/g erreicht.

### Pharmakologische Eigenschaften:

GHB löst bei einer Dosis von 35 bis etwa 90 mg/kg Körpergewicht eine hypnotische, bei Dosierungen über 100 mg/kg Körpergewicht eine narkotische Wirkung aus. Da Somsanit unterhalb 90 mg/kg Körpergewicht keine analgetischen Eigenschaften besitzt, muß es zur Erzielung einer ausreichenden Anaesthesie bei chirurgischen Eingriffen mit Analgetika, Neuroleptika oder mit einer unterschwelligen Barbiturat-Dosis kombiniert werden. Bei internen Indikationen hingegen (z.B. Schlaftherapie, Carcinom-Endzustand) läßt sich GHB auch als "Mononarkotikum" verwenden.

Die Untersuchungen von A.E. USPENSKIJ haben gezeigt, daß 4-hydroxybuttersaures Natrium die polysynaptischen Reflexe hemmt, während die monosynaptischen Reflexe unbeeinflußt bleiben, selbst bei Dosierungen bis zu 2 g/kg Katze.

Einige besondere Eigenschaften, die das GHB von anderen Anästhetika unterscheidet, sind erwähnenswert: der Lidreflex erlischt, die Augenlider erschlaffen, die Augen bleiben jedoch häufig halb offen. Der Cornealreflex ist im allgemeinen erhalten. Das Einschlafen erfolgt langsam, das Erwachen relativ schnell. In Abhängigkeit von der Dosis beträgt die Wirkungszeit 1-2 Std. Die mit GHB erzielte Narkose gleicht nach den Untersuchungen von FISHBEIN und BESSMAN weitgehend dem physiologischen Schlaf. Im allgemeinen findet sich eine Vertiefung der Atmung mit Erhöhung der Amplitude und Abnahme der Atemfrequenz. Die Empfindlichkeit des Atemzentrums gegenüber einem Kohlensäurereiz bleibt erhalten. GHB wirkt selbst also nicht atemdepressiv, kann jedoch die atemdepressive Wirkung anderer Anaesthetika eventuell potenzieren.

In Ausnahmefällen tritt für eine kurze Zeitspanne, insbesondere während der Aufwachphase, eine typische periodische Atmung auf. Störungen des Säure-Basen-Haushaltes ließen sich in diesen Phasen jedoch nicht nachweisen, eine Normalisierung tritt nach kurzer Zeit ohne therapeutische Maßnahmen ein.

Nach Verabreichung von GHB ist häufiger ein Blutdruckanstieg zu beobachten. In den bisher vorliegenden Untersuchungen konnte auch bei Verwendung hoher Dosen keine direkte depressive Wirkung auf das Myokard nachgewiesen werden. Untersuchungen, die an Ratten erfolgten, ermöglichen eine Aussage über den Abbau des GHB im Organismus, 97 % des markierten Kohlenstoffs (C¹⁴) ließen sich innerhalb von 2 Std. als CO₂ in der Ausatemluft nachweisen.

Zu erwähnen bleibt schließlich noch, daß der Elektrolythaushalt unter einer GHB-Narkose nicht wesentlich verändert wird. Es kommt lediglich zu einer Kaliumverschiebung vom extrazellulären in den intrazellulären Flüssigkeitsraum. Diese Verschiebung erreicht jedoch nie ein bedrohliches Ausmaß.

### Bedeutung der Erfindung:

Bezüglich der bisher beobachteten geringfügigen Nebenwirkungen war stets das Augenmerk auf die spezifische Wirkung der GHB gerichtet und das Kation Natrium blieb unberücksichtigt, da es lediglich als physiologischer Ladungsträger die Wasserlöslichkeit verbessert und der klinische Stellenwert als galenische Komponente gering eingestuft wurde.

Mit der zunehmenden Bedeutung der GHB auch bei geschlossenen Schädel-Hirn-Traumen, sowie im Bereich der oralen Anwendung zur Therapie des Alkoholentzugs, der Narkolepsie und als Schlafmittel kommt den Nebenwirkungen größere Bedeutung zu. Die kritischen Parameter sollen in Stichworten wie folgt skizziert werden:
bei i.v. Gabe:
# intrakranieller Druckanstieg
# Elektrolytverschiebung
# cave Niereninsuffizienz
# klonische Muskelzuckungen
bei oraler Gabe:
# Übelkeit
# Geschmack
# Diarrhoe
# Compliance
Der Erfindung liegt daher die Aufgabe zugrunde, die Nebenwirkungen weiter zu reduzieren.

Dazu schlägt die Erfindung neue wasserlösliche Derivate der 4-Hydroxy-Buttersäure (GHB) mit verbesserten klinischen und pharmakologischen Eigenschaften gemäß Ansprüchen 1 und 2 vor. Die Herstellung der Salze und Amide gelingt entsprechend den Ansprüchen 14 und 15, während die Erfindung Mittel zu speziellen Indikationen gemäß den Ansprüchen 16 und 17 beinhaltet.

Die erfindungsgemäßen Verbindungen sind geeignet, beispielsweise die Wirksamkeit von Antibiotika weiter zu steigern.

Bevorzugte Beispiele der erfindungsgemäßen Salze sind in den Ansprüchen 2 bis 8 angegeben. Bevorzugte Beispiele der erfindungsgemäßen Amide sind in den Ansprüchen 9 bis 14 genannt.

Zur Herstellung der erfindungsgemäßen Salze wird einer wässrigen Lösung von gamma-Butyrolacton ein Aminopolyalkohol der angegebenen Art zugegeben, die wässrige Lösung bis zur Einstellung ihres pH-Wertes auf etwa 7,5 schwach erwärmt und anschließend das Volumen der erhaltenen Lösung durch Wasserzugabe auf die gewünschte Konzentration von 4-Hydroxy-Buttersäure eingestellt.

Zur Herstellung der Amide der gamma-Hydroxybuttersäure läßt man gamma-Butyrolacton mit einem primären oder sekundären Aminoalkohol oder einem Morpholinderivat in einem geeigneten Lösungsmittel, bevorzugt einem niederen Alkohol bei Temperaturen zwischen 40°C und 120°C miteinander reagieren. Nach dem Abdestillieren des Lösungsmittels wird das gewünschte Amid erhalten.

Die erhaltenen wässrigen Lösungen der erfindungsgemäßen Salze und/oder Amide sind als parenterale Zubereitungsformen zur Narkose im Rahmen der Anästhesie, zur Schlafinduktion und im Rahmen der Langzeitsedierung wirksam und können als orale Zubereitungsformen bei der Therapie des Alkoholentzugs, der Kataplexie, der Narkolepsie oder des Symptomenkomplexes Schlafstörungen erfolgreich eingesetzt werden. Bei einer pharmakologischen Verwendung der erfindungsgemäßen Buttersäurederivate treten die oben angesprochenen, auf das Natrium im Na-Salz der GHB zurückführenden Nebenwirkungen im wesentlichen nicht mehr auf.

### Beispiel 1:

### 1-Desoxy-1-methylamino-D-Gucitol 4-Hydroxybutyrat

500 ml (565 g, 6,56 Mol) gamma-Butyrolacton werden in 1,8 l Wasser gegeben. Zu der erhaltenen Lösung fügt man 1,28 kg (6,56 Mol) Methylglucamin hinzu. Nun wird 8 h lang auf 60xC erwärmt. Der pH-Wert sinkt dabei von anfänglich 11 auf 7,5. Nun wird das Volumen der erhaltenen Lösung durch Zugabe von Wasser (ca. 200 ml) auf 3,4 l eingestellt um eine Konzentration von 2 g gamma-Hydroxybuttersäure in 10 ml Lösung zu erreichen.

### Beispiel 2:

### Trishydroxymethyl-aminomethan-4-hydroxybutyrat

500 ml (565 g, 6,56 Mol) gamma-Butyrolacton werden in 1,8 l Wasser gegeben. Zu der erhaltenen Lösung fügt man 795 g (6,56 Mol) Trishydroxymethylmethylamin hinzu. Nun wird 8h lang auf 60xC erwärmt. Der pH-Wert sinkt dabei von anfänglich 10,5 auf 7,5. Nun wird das Volumen der erhaltenen Lösung durch Zugabe von Wasser auf 3,4 l eingestellt um eine Konzentration von 2 g gamma-Hydroxybuttersäure in 10 ml Lösung zu erreichen.

### Beispiel 3:

### 1-Desoxy-1-(N-4-hydroxybutyroyl-N-methylamino)-D-Gucitol

500 ml (565 g, 6,56 Mol) gamma-Butyrolacton werden in 2,0 l Methanol gegeben. Zu der erhaltenen Lösung fügt man 1,28 kg (6,56 Mol) Methylglucamin hinzu. Nun wird 16 h lang unter Rückfluß zum Sieden erhitzt. Die erhaltene Lösung wird zur Trockene eingeengt. Der dabei zunächst erhaltene farblose Sirup kristallisiert nach einigen Tagen bei Raumtemperatur zu einem weißen Feststoff. (Schmp. 78°-79°C)

### Beispiel 4:

### N-(2-Hydroxyethyl)-4-hydroxybutyramid

Zu einer Lösung von 88g gamma-Butyrolacton in 120ml wird eine Lösung von 61g Ethanolamin in 70 ml Methanol getropft. Es wird 8h unter Rückfluß erhitzt und anschließend zur Trockene eingeengt. Das erhaltene viskose Öl kristallisiert nach längerem stehen. Die Substanz wird aus Aceton umkristallisiert und im Vakuum getrocknet. (Weißer Feststoff Schmp. 55-56°C)

## Patentansprüche

1. Wasserlösliche Derivate der 4-Hydroxy-Buttersäure (GHB) mit der Formel 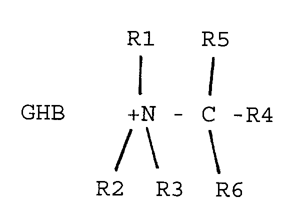 wobei R1, R2 und R3 H-, CH₃-, C₂H₅-,CH₂OH-CH₂- oder CH₂OH-CHOH-CH₂-Reste bedeuten oder mit dem Stickstoff ein Heterocycloalkan mit 4 oder 5 Kohlenstoffatomen bilden,
wobei ferner R4 ein Alkohol oder Polyalkohol mit 1 bis 5 Kohlenstoffatomen und 1 bis 5 Sauerstoffatomen von linearer, verzweigter oder ringförmiger Struktur ist und R1 und R4 zu einem cyclischen Ether miteinander kondensiert sein können, und
R5 und R6 unabhängig voneinander H- oder CH₂OH-sind.

2. Wasserlösliche Derivate der 4-Hydroxy-Buttersäure mit der Formel 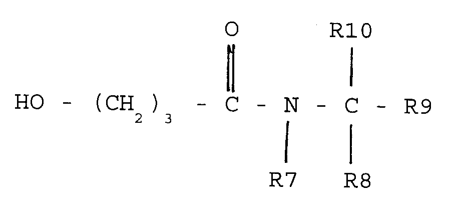 wobei R7 H-, CH₃-, C₂H₅-, CH₂OH-CH₂- oder CH₂OH-CHOH-CH₂- Reste, R9 einen Alkohol oder Polyalkohol mit 1 bis 5 C-Atomen und 1 bis 5 Sauerstoffatomen von linearer, verzweigter oder ringförmiger Struktur, R8 und R10, unabhängig voneinander, H- oder CH₂OH bedeuten, wenn R7 eine Alkylgruppe ist und R8=H und R10=CH2OH sind , wenn R7 Wasserstoff ist, und wobei R7 und R9 auch zu einem zyklischen Ether kondensiert sein können.

3. 2-Desoxy-2-Methylaminoglucose 4-Hydroxybutyrat

4. 1-Methylaminopropandiol 4-Hydroxybutyrat

5. 2-Methylaminopropandiol 4-Hydroxybutyrat

6. 1-Methylaminobutantriol 4-Hydroxybutyrat

7. 2-Methylaminobutantriol 4-Hydroxybutyrat

8. 1-Desoxy-1-Methylamino-D-Glucitol 4-Hydroxybutyrat

9. Trishydroxymethylaminomethan 4-Hydroxybutyrat

10. 1-Desoxy-1-(N-4-hydroxybutyroyl-N-methylamino)-D-Gucitol

11. N-2-Hydroxyethyl-N-methyl-4-hydroxybutyramid

12. N-(1-hydroxymethyl-2-hydroxyethyl)-4-hydroxy butyramid

13. 4-Hydroxybuttersäuremorpholid

14. Herstellung der Salze nach einem der vorstehenden Ansprüche, dadurch gekennzeichnet, daß zu einer wässrigen Lösung von gamma-Butyrolacton ein Aminoalkohol der angegebenen Art zugesetzt wird, die Lösung bis zum Erreichen ihres pH-Wertes von etwa 7,5 schwach erwärmt und anschließend durch Wasserzugabe auf die gewünschte Konzentration der gamma-Hydroxybuttersäure eingestellt wird.

15. Herstellung der Amide der gamma-Hydroxybuttersäure dadurch gekennzeichnet, daß man gamma-Butyrolacton mit einem Morpholinderivat in einem geeigneten Lösungsmittel, bevorzugt einem niederen Alkohol miteinander reagieren läßt.

16. Mittel zur Narkose im Rahmen der Anästhesie oder zur Schlafinduktion oder im Rahmen der Langzeitsedierung enthaltend Derivate der 4-Hydroxy-Buttersäure (GHB) mit den Formeln I und II 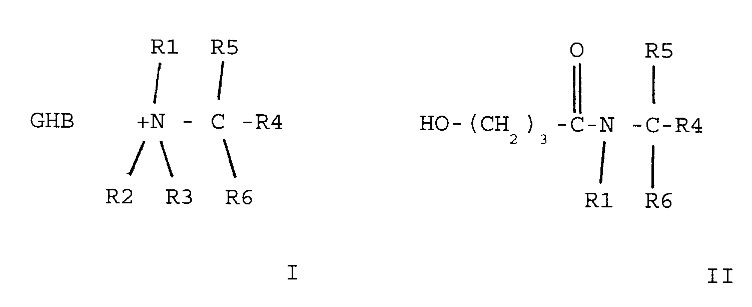 wobei R1, R2 und R3 H-, CH₃-, C₂H₅-,CH₂OH-CH₂- oder CH₂OH-CHOH-CH₂-Reste bedeuten oder mit dem Stickstoff ein Heterocycloalkan mit 4 oder 5 Kohlenstoffatomen bilden,
wobei ferner R4 ein Alkohol oder Polyalkohol mit 1 bis 5 Kohlenstoffatomen und 1 bis 5 Sauerstoffatomen von linearer, verzweigter oder ringförmiger Struktur ist und R1 und R4 zu einem cyclischen Ether miteinander kondensiert sein können, und R5 und R6 unabhängig voneinander H- oder CH₂OH- sind, in wässrigen Lösungen als parenterale Zubereitungsform.

17. Mittel zur Therapie des Alkoholentzugs oder zur Therapie der Kataplexie oder Narkoplepsie oder zur Therapie des Symptomenkomplexes Schlafstörungen, enthaltend Derivate der 4-Hydroxy-Buttersäure 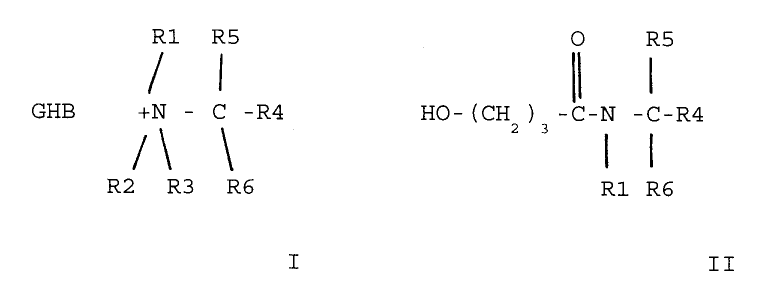 wobei R1, R2 und R3 H-, CH₃-, C₂H₅-,CH₂OH-CH₂- oder CH₂OH-CHOH-CH₂-Reste bedeuten oder mit dem Stickstoff ein Heterocycloalkan mit 4 oder 5 Kohlenstoffatomen bilden,
wobei ferner R4 ein Alkohol oder Polyalkohol mit 1 bis 5 Kohlenstoffatomen und 1 bis 5 Sauerstoffatomen von linearer, verzweigter oder ringförmiger Struktur ist und R1 und R4 zu einem cyclischen Ether miteinander kondensiert sein können, und
R5 und R6 unabhängig voneinander H- oder CH₂OH-sind, als orale Zubereitungsform.

## Claims

1. Water-soluble derivatives of 4-hydroxy butyric acid (GHB) of the formula 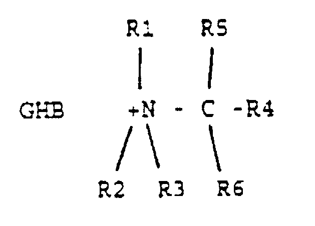 in which **R1**, **R2** and **R3** represent H-, CH₃-, C₂H₅-, CH₂OH-CH₂- or CH₂OH-CHOH-CH₂- residues or form a heterocycloalkane [*sic*] having 4 or 5 carbon atoms with the nitrogen,
in which in addition **R4** is an alcohol or polyalcohol having from 1 to 5 carbon atoms and from 1 to 5 oxygen atoms of linear, branched or cyclic structure, and **R1** and **R4** can be condensed with each other to form a cyclic ether, and
**R5** and **R6** are H- or CH₂OH- independently of each other

2. Water-soluble derivatives of 4-hydroxy butyric acid of the formula 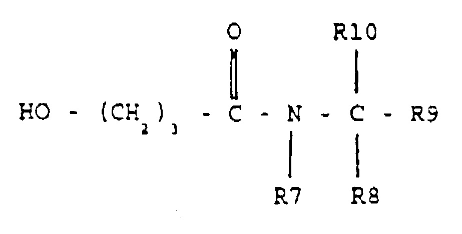 in which **R7** represents H-, CH₃-, C₂H₅-, CH₂OH-CH₂- or CH₂OH-CHOH-CH₂-residues [*sic*], **R9** represents an alcohol or polyalcohol having from 1 to 5 carbon atoms and from 1 to 5 oxygen atoms of linear, branched or cyclic structure, **R8** and **R10** represent H- or CH₂OH independently of each other, when **R7** is an alkyl group and **R8**=H and **R10**=CH₂OH when **R7** is hydrogen, and
in which **R7** and **R9** can also be condensed to form a cyclic ether.

3. 2-deoxy-2-methylaminoglucose 4-hydroxybutyrate.

4. 1-methylaminopropanediol 4-hydroxybutyrate.

5. 2-methylaminopropanediol 4-hydroxybutyrate.

6. 1-methylaminobutanetriol 4-hydroxybutyrate.

7. 2-methylaminobutanetriol 4-hydroxybutyrate.

8. 1-deoxy-1-methylamino-D-glucitol 4-hydroxybutyrate.

9. Tris-hydroxymethylaminomethane 4-hydroxybutyrate.

10. 1-deoxy-1-(N-4-hydroxybutyrol-N-methylamino)-D-glucitol.

11. N-2-hydroxyethyl-N-methyl-4-hydroxybutyramide.

12. N-(1-hydroxymethyl-2-hydroxyethyl)-4-hydroxybutyramide.

13. 4-hydroxy butyric acid morpholide.

14. Preparation of the salts according to one of the preceding Claims, **characterized in that** an aminoalcohol of the type indicated is added to an aqueous solution of γ butyrolactone, and the solution is gently heated until its pH value reaches approximately 7·5 and the solution is then set to the desired concentration of the γ hydroxy butyric acid by the addition of water.

15. Preparation of the amides of γ hydroxy butyric acid, **characterized in that** γ butyrolactone is allowed to react with a morpholine derivative in a suitable solvent, preferably a lower alcohol.

16. An agent for narcosis in the context of anaesthesia or sleep induction or in the context of long-term sedation containing derivatives of 4-hydroxy butyric acid (GHB) of the formulae I and II 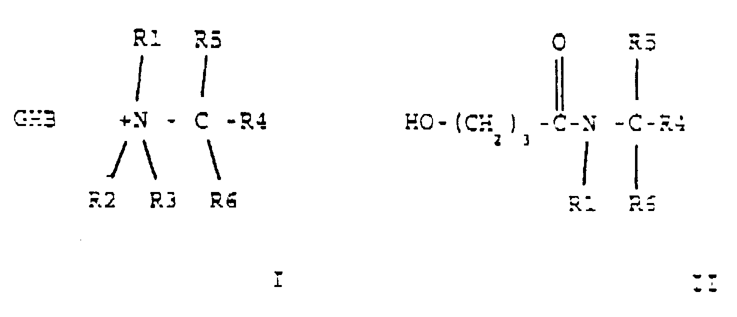 in which **R1**, **R2** and **R3** represent H-, CH₃-, C₂H₅-, CH₂OH-CH₂- or CH₂OH-CHOH-CH₂- residues or form a heterocycloalkane [*sic*] having 4 or 5 carbon atoms with the nitrogen,
in which in addition **R4** is an alcohol or polyalcohol having from 1 to 5 carbon atoms and from 1 to 5 oxygen atoms of linear, branched or cyclic structure, and **R1** and **R4** can be condensed with each other to form a cyclic ether, and **R5** and **R6** are H- or CH₂OH- independently of each other, in aqueous solutions as a parenteral form of preparation.

17. An agent for the therapy of alcohol withdrawal or for the therapy of cataplexy or narcolepsy or for the therapy of the symptom complex of sleep disorders, containing derivatives of 4-hydroxy butyric acid 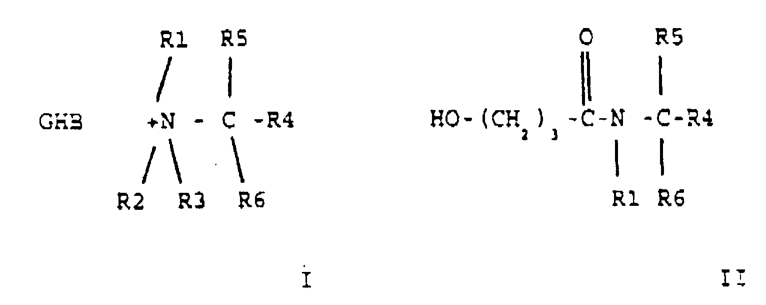 in which **R1**, **R2** and **R3** represent H-, CH₃-, C₂H₅-, CH₂OH-CH₂- or CH₂OH-CHOH-CH₂- residues or form a heterocycloalkane [*sic*] hating 4 or 5 carbon atoms with the nitrogen,
in which in addition **R4** is an alcohol or polyalcohol having from 1 to 5 carbon atoms and from 1 to 5 oxygen atoms of linear, branched or cyclic structure, and **R1** and **R4** can be condensed with each other to form a cyclic ether, and
**R5** and **R6** are H- or CH₂OH- independently of each other, as an oral form of preparation.

## Revendications

1. Dérivés solubles dans l'eau de l'acide 4-hydroxybutirique (GHB) de formule 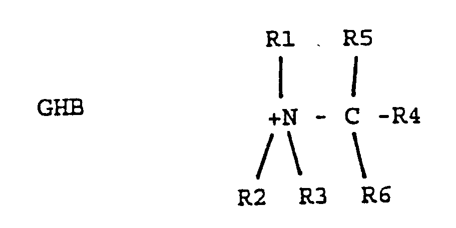 dans laquelle R1, R2 et R3 représentent des radicaux H-, CH₃-, C₂H₅-, CH₂OH-CH₂- ou CH₂OH-CHOH-CH₂ - ou bien forment avec l'atome d'azote un hétérocycloalcane comportant 4 ou 5 atomes de carbone,
dans laquelle de plus R4 représente un alcool ou un polyalcool comportant de 1 à 5 atomes de carbone et comportant de 1 à 5 atomes d'oxygène, de structure linéaire, ramifiée ou cyclique, et R1 et R4 peuvent être condensés ensemble pour former un éther cyclique, et
R5 et R6 représentent, indépendamment l'un de l'autre, H- ou CH₂OH-.

2. Dérivés solubles dans l'eau de l'acide 4-hydroxybutirique de formule 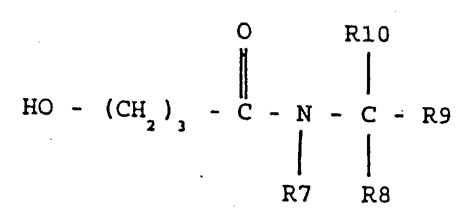 dans laquelle R7 représente un radical H-, CH₃-, C₂H₅-, CH₂ OH-CH₂- ou CH₂OH-CHOH-CH₂ -, R9 représente un alcool ou un polyalcool comportant de 1 à 5 atomes de carbone et comportant de 1 à 5 atomes d'oxygène, de structure linéaire, ramifiée ou cyclique, R8 et R10, indépendamment l'un de l'autre, représentent un reste H-ou CH₂OH, lorsque R7 représente un groupe alkyle et R8 = H et R10 = CH₂OH, lorsque R7 représente un atome d'hydrogène, et dans laquelle R7 et R9 peuvent également être condensés en un éther cyclique.

3. 4-hydroxybutyrate de 2-désoxy-2-méthylaminoglucose

4. 4-hydroxybutyrate de 1-méthylaminopropanediol

5. 4-hydroxybutyrate de 2-méthylaminopropanediol

6. 4-hydroxybutyrate de 1-méthylaminobutanetriol

7. 4-hydroxybutyrate de 2-méthylaminobutanetriol

8. 4-hydroxybutyrate de 1-désoxy-1-méthylamino-D-glucitol

9. 4-hydroxybutyrate de trishydroxyméthylaminométhane

10. 1-désoxy-1-(N-4-hydroxybutyroyl-N-méthylamino)-D-glucitol

11. N-2-hydroxyéthyl-N-méthyl-4-hydroxybutyramide

12. N-(1-hydroxyméthyl-2-hydroxyéthyl)-4-hydroxybutyramide

13. morpholide de l'acide 4-hydroxybutirique

14. Procédé de préparation des sels selon l'une quelconque des revendications précédentes, **caractérisé** en ce que l'on ajoute un aminoalcool du type indiqué à une solution aqueuse de gamma-butyrolactone, on réchauffe faiblement la solution jusqu'à atteindre un pH d'environ 7,5, et ensuite on règle la solution à la concentration souhaitée d'acide gamma-hydroxybutirique par addition d'eau.

15. Procédé de préparation des amides de l'acide gamma-hydroxybutirique, **caractérisé** en ce que l'on fait réagir la gamma-butyrolactone avec un dérivé de morpholine dans un solvant approprié, de préférence un alcool inférieur.

16. Agent pour la narcose dans le cadre de l'anesthésie ou pour l'induction du sommeil, ou dans le cadre de traitements de longue durée sous sédatifs, contenant des dérivés de l'acide 4-hydroxybutirique (GHB) de formules I et II 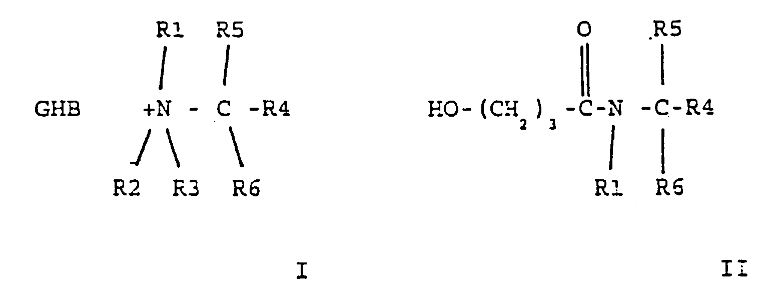 dans lesquelles R1, R2 et R3 représentent des radicaux H-, CH₃-, C₂H₅-, CH₂ OH-CH₂- ou CH₂OH-CHOH-CH₂- ou forment avec l'atome d'azote un hétérocycloalcane comportant 4 ou 5 atomes de carbone.
dans lesquelles de plus R4 représente un alcool ou un polyalcool comportant de 1 à 5 atomes de carbone et comportant de 1 à 5 atomes d'oxygène, de structure linéaire, ramifiée ou cyclique, et R1 et R4 peuvent être condensés ensemble pour former un éther cyclique, et R5 et R6 représentent indépendamment l'un de l'autre un reste H- ou CH₂OH-, en solutions aqueuses comme préparations pour administration parentérale.

17. Agent pour la thérapie de la désintoxication alcoolique, ou bien pour la thérapie de la cataplexie ou de la narcoplepsie, ou bien pour la thérapie du complexe des symptômes des troubles du sommeil, contenant des dérivés de l'acide 4-hydroxybutirique de formules 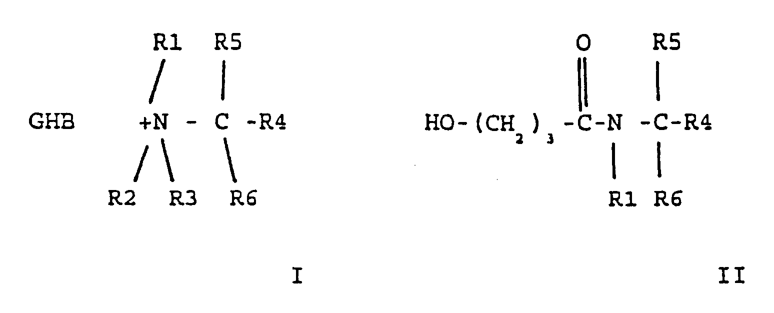 dans lesquelles R1, R2 et R3 représentent des radicaux H-, CH₃-, C₂H₅-, CH₂ OH-CH₂- ou CH₂OH-CHOH-CH₂- , ou forment avec l'atome d'azote un hétérocycloalcane comportant 4 ou 5 atomes de carbone,
dans lesquelles de plus R4 représente un alcool ou polyalcool comportant de 1 à 5 atomes de carbone et comportant de 1 à 5 atomes d'oxygène, de structure linéaire, ramifiée ou cyclique, et R1 et R4 peuvent être condensés ensemble en un éther cyclique, et
R5 et R6 représentent indépendamment l'un de l'autre un reste H- ou CH₂OH- , sous la forme d'une préparation pour administration orale.
